# EUROPEAN PATENT APPLICATION

(11) **EP 3 520 852 A1**
(43) Date of publication of application: **07.08.2019**
(21) Application number: 17855556.1
(22) Date of filing: 31.08.2017
(51) Int. Cl.: A61M 25/092, A61M 25/00, A61M 25/14

(54) **CATHETER**

(30) Priority: 30.09.2016 JP 2016194360
(71) Applicant: Nipro Corporation, Osaka-shi, Osaka 531-8510 (JP)
(72) Inventor: SASAKI, Miyoko, Sendai-shi Miyagi 981-3132 (JP); YOSHIZAWA, Kiyofumi, Osaka-shi Osaka 531-8510 (JP)
(74) Representative: Kraus & Weisert Patentanwälte PartGmbB
(86) International application number: PCT/JP2017/031335
(87) International publication number: WO 2018/061598

(57) **Abstract**

Provided is a catheter with a novel structure which is able to allow its distal end portion to deform to curve more effectively in response to an operation from an outside. In a catheter 10, a control lumen 24 extending in a lengthwise direction is provided to an elongated shaft member 33, a control wire 54 is inserted through the control lumen 24, a fixing part 57 to which a distal end of the control wire 54 is fixed is provided to the shaft member 33, and an exposed part 60 is provided to a distal end portion of the control wire 54 over a predetermined length so as to be exposed through a window part 58 provided to a peripheral wall 28 of the control lumen 24 to a separate internal space 12 in the shaft member 33.

## Description

### TECHNICAL FIELD

The present invention relates to a catheter inserted percutaneously into a somatic lumen and used for observation or treatment of a lesion, and the like.

### BACKGROUND ART

Conventionally, tubular members such as catheters have been known as one type of medical instruments for performing examination, treatment, and the like on somatic tubular tissues such as blood vessels and tubular organs. Specifically, catheters are used in the treatment of PCTA (percutaneous coronary transluminal angioplasty), PTCR (percutaneous transluminal coronary recanalization), PTCD (percutaneous transhepatic cholangio drainage), PEIT and the like.

Incidentally, the somatic lumen often bends, curves, and branches, and when inserting a tubular member such as a catheter, it is necessary to deform the tubular member to curve corresponding to the curved shape of the somatic lumen and the like. Whereas it is possible to deform the tubular member to curve by pressing it against the inner wall surface of the somatic lumen or the like, if the tubular member is strongly pressed against the inner wall surface, there is a risk of the somatic lumen being damaged. Thus, it is preferable that the tubular member has a structure such that the user can appropriately deform the tubular member to curve corresponding to the shape of the somatic lumen.

In light of that, Japanese Domestic Publication of International Patent Application No. JP-A-2014-521447 (Patent Document 1) proposes a catheter whose distal end portion can be deformed to curve by a user's operation. That is, in the inside of the catheter described in Patent Document 1, provided is a steering element such as a wire having one end fixed to the distal end portion of the catheter. By pushing and pulling the other end of the steering element in the lengthwise direction of the catheter from the outside, it is possible for the user to deform the distal end portion of the catheter to curve.

However, with the catheter described in Patent Document 1, the steering element such as a wire is only inserted into a steering element lumen extending in a tubular shape in the lengthwise direction of the catheter to have its distal end fixed to the catheter. Therefore, for example, during pushing operation of the steering element, the steering element may come into contact with the inner surface of the peripheral wall of the steering element lumen, posing a risk of adversely affecting the curving deformation at the distal end portion of the catheter, or requiring a large operating force due to the frictional resistance.

### BACKGROUND ART DOCUMENT

### PATENT DOCUMENT

Patent Document 1: JP-A-2014-521447

### SUMMARY OF THE INVENTION

### PROBLEM THE INVENTION ATTEMPTS TO SOLVE

The present invention has been developed in view of the above-described matters as the background, and it is an object of the present invention to provide a catheter with a novel structure which is able to allow its distal end portion to deform to curve more effectively in response to an operation from the outside.

### MEANS FOR SOLVING THE PROBLEM

The above and/or optional objects of this invention may be attained according to at least one of the following preferred embodiments of the invention. The following preferred embodiments and/or elements employed in each preferred embodiment of the invention may be adopted at any possible optional combinations.

A first preferred embodiment of the present invention provides a catheter comprising: an elongated shaft member including at least one control lumen extending in a lengthwise direction; at least one control wire inserted through the control lumen; and at least one fixing part to which a distal end of the control wire is fixed, the fixing part being provided to the shaft member, wherein at least one window part is provided to a peripheral wall of the control lumen, and an exposed part is provided to a distal end portion of the control wire over a predetermined length, the exposed part being exposed through the window part to a separate internal space in the shaft member.

According to the catheter structured following the present preferred embodiment, the control wire is exposed to the separate internal space. Thus, during operation of the control wire, the degree of freedom of deformation of the control wire is sufficiently ensured, and sliding contact resistance of the control wire with respect to the peripheral wall of the control lumen may also be reduced. Therefore, the degree of freedom in setting the deforming force that can be exerted on the catheter by operating the control wire is improved, and ease of operation of the control wire may be improved.

Furthermore, the distal end portion of the control wire is exposed to the separate internal space over a predetermined length. This may obtain a sufficient length dimension for flexural deformation of the control wire, and the contact between the control wire and the peripheral wall of the control lumen during the flexural deformation may be more effectively prevented.

A second preferred embodiment of the present invention provides the catheter according to the first preferred embodiment, wherein the separate internal space comprises a separate lumen extending in the lengthwise direction of the shaft member.

According to the catheter structured following the present preferred embodiment, by placing the control lumen and the separate lumen in communication with each other, the window part is formed by such a communicating portion and the control wire is exposed to the separate internal space. Thus, the structure of the catheter can be further simplified.

A third preferred embodiment of the present invention provides the catheter according to the second preferred embodiment, wherein the separate lumen comprises a main lumen extending with a cross section larger than that of the control lumen.

According to the catheter structured following the present preferred embodiment, since the control wire is exposed to the main lumen having the large cross section in the shaft member, when the control wire is pushed in, the control wire is allowed to undergo a greater flexural deformation. This makes it possible to more effectively avoid the contact between the control wire and the peripheral wall of the control lumen during the flexural deformation, while more effectively achieving the degree of freedom in setting the operating force such as bending exerted on the shaft member through the control wire.

A fourth preferred embodiment of the present invention provides the catheter according to any of the first to third preferred embodiments, wherein the window part is provided at a position away from the fixing part of the shaft member by 10 to 50 mm.

According to the catheter structured following the present preferred embodiment, in the catheter lumen, cushioning action is exerted by the deformation of the curved portion with respect to the operating force exerted on the control wire. Thus, for example, in the case where the catheter comprises a thrombus suction catheter, suction of a thrombus by bending the distal end and the like can be effectively realized.

If the remote distance between the window part and the fixing part is less than 10 mm, the region of bending deformation at the distal end of the catheter is too small (namely, the amount of deformation at the distal end of the catheter is too small), and the distal end of the catheter cannot be brought sufficiently close to the inner wall of the lumen such as the blood vessel. Thus, for example, when the catheter comprises a suction catheter, there is a risk that a thrombus or the like adhering to the inner wall of the blood vessel may not be sufficiently suctioned. Besides, if the remote distance between the window part and the fixing part is larger than 50 mm, the starting point of bending in the catheter is too far from the distal end of the catheter. Thus, the curvature of the bent portion of the catheter with respect to the curvature of the lumen is too small, and there is a risk that it may be difficult to deform the catheter into a shape corresponding to the curved shape of the lumen. In addition, the amount of operation of the control wire, that is, the amount of operation load exerted on the control wire, becomes too large, posing a risk of increasing stress applied to the blood vessel.

A fifth preferred embodiment of the present invention provides the catheter according to any of the first to fourth preferred embodiments, wherein the fixing part is provided at a position away from a distal end of the shaft member by 10 to 40 mm.

According to the catheter structured following the present preferred embodiment, the distal end of the catheter is appropriately brought into contact with the treatment site of the inner wall of the lumen such as the blood vessel. Thus, for example, when the catheter comprises a thrombus suction catheter, suction of a thrombus by bending the distal end and the like can be effectively realized. If the remote distance between the distal end of the shaft member and the fixing part is less than 10 mm, the region of bending deformation at the distal end of the catheter is too small (namely, the amount of deformation at the distal end of the catheter is too small), and the distal end of the catheter cannot be brought sufficiently close to the inner wall of the lumen. Thus, for example, when the catheter comprises a suction catheter, there is a risk that a thrombus or the like adhering to the inner wall of the blood vessel may not be sufficiently suctioned. Besides, if the remote distance between the distal end of the shaft member and the fixing part is larger than 40 mm, the starting point of bending in the catheter is too far from the distal end of the catheter. Thus, the curvature of the bent portion of the catheter with respect to the curvature of the lumen is too small, and there is a risk that it may be difficult to deform the catheter into a shape corresponding to the curved shape of the lumen. In addition, the amount of operation of the control wire, that is, the amount of operation load exerted on the control wire becomes too large, posing a risk of increasing stress applied to the blood vessel.

A sixth preferred embodiment of the present invention provides the catheter according to any of the first to fifth preferred embodiments, further comprising a controller provided on a proximal end side of the control wire, the controller being configured to exert an operating force in the lengthwise direction from an outside.

According to the catheter structured following the present preferred embodiment, since the controller is provided, the user can easily exert the operating force on the control wire from the outside by grasping and operating the controller.

A seventh preferred embodiment of the present invention provides the catheter according to any of the first to sixth preferred embodiments, wherein the shaft member is reinforced by a braided wire embedded as an intermediate layer thereof.

According to the catheter structured following the present preferred embodiment, the embedded region of the braided wire is reinforced. Thus, in the case where the braided wire is embedded in the shaft member, for example, the shaft member on the radially outer side of the exposed part of the control wire, the flexibility of the catheter is guaranteed, and in addition, kink resistance, torque transmissibility, pushability, etc. of the entire catheter can be improved. Although the embedded region of the braided wire is not limited in any way, the region is preferably provided to, for example, inside the peripheral wall of the main lumen, the control lumen, and the like.

An eighth preferred embodiment of the present invention provides the catheter according to any one of the first to seventh preferred embodiments, wherein a radially inner surface of either one of the control lumen and the separate internal space is formed of a material different from that of a main body of the shaft member.

According to the catheter structured following the present preferred embodiment, with the shaft member, by making the material of the radially inner surface of the control lumen or the separate internal space separated from the control lumen and the material of the other portions different from each other, various types of characteristics can be imparted to the catheter. That is, for example, by forming the peripheral wall of the control lumen with a material softer than the other portions, it is also possible to make the catheter easier to deform to curve. Besides, in the present preferred embodiment, it is also possible to provide a coating layer on the inner surface of the control lumen. For example, by forming the coating layer provided on the inner surface of the control lumen with a material having a small friction coefficient, sliding resistance of the control wire with respect to the control lumen may be reduced.

Further, according to the catheter structured following any of the ninth to fourteenth preferred embodiments described below, it is possible to deform the catheter to curve in a desired direction and into a desired shape by operating the control wire.

A ninth preferred embodiment of the present invention provides the catheter according to any of the first to eighth preferred embodiments, wherein the window part is provided further on a proximal end side than the fixing part in the lengthwise direction of the shaft member.

A tenth preferred embodiment of the present invention provides the catheter according to any of the first to eighth preferred embodiments, wherein the window part is provided further on a distal end side than the fixing part in the lengthwise direction of the shaft member.

An eleventh preferred embodiment of the present invention provides the catheter according to any of the first to tenth preferred embodiments, wherein the at least one control wire comprises a plurality of control wires, the at least one fixing part comprises a plurality of fixing parts provided correspondingly to the plurality of control wires, and positions of the fixing parts are different from each other in a circumferential direction.

According to the catheter structured following the present preferred embodiment, by providing a plurality of control wires, it is also possible to deform the catheter to curve in more directions.

A twelfth preferred embodiment of the present invention provides the catheter according to the eleventh preferred embodiment, wherein the at least one control lumen through which the control wires are inserted comprises a plurality of control lumens provided correspondingly to the plurality of control wires.

According to the catheter structured following the present preferred embodiment, the plurality of control wires are inserted into the respective separate control lumens. Thus, mutual interference between the control wires can be effectively prevented.

A thirteenth preferred embodiment of the present invention provides the catheter according to any of the first to twelfth preferred embodiments, wherein the at least one window part comprises a plurality of window parts spaced away from each other in the lengthwise direction of the shaft member.

A fourteenth preferred embodiment of the present invention provides the catheter according to any of the first to thirteenth preferred embodiments, wherein the at least one control lumen comprises a plurality of control lumens provided at different positions in either one of a circumferential direction and the lengthwise direction of the shaft member.

In addition, the catheter according to the present invention preferably comprises a suction catheter for suctioning a thrombus and the like in a blood vessel.

Besides, the suction catheter is preferably used for a lower-limb blood vessel. According to the catheter structured following the present preferred embodiment, in the case of a large blood vessel diameter such as a lower-limb blood vessel in which the inner diameter of the blood vessel is 4 to 15 mm, it is possible to effectively suction a thrombus and the like by bending the distal end of the catheter. The lower-limb blood vessels are preferably iliac artery and vein, femoral artery and vein, popliteal (below-knee) artery and vein, and the like.

### EFFECT OF THE INVENTION

According to the catheter structured following the present invention, during the pushing operation of the control wire, the contact between the control wire and the control lumen is effectively avoided. This makes it possible to minimize the influence due to the contact therebetween, thereby more reliably deforming the catheter to curve.

### BRIEF DESCRIPTION OF THE DRAWINGS

FIG. 1 is a front view showing an entirety of a catheter according to a first embodiment of the present invention.
FIG. 2 is a vertical cross sectional view schematically showing a principal part of the catheter shown in FIG. 1.
FIG. 3 is a cross sectional view taken along line 3-3 of FIG. 2.
FIG. 4 is a cross sectional view taken along line 4-4 of FIG. 2.
FIG. 5 is a view suitable for explaining curving deformation of the catheter when an operating force in a pulling direction is exerted on a control wire in the catheter shown in FIG. 1.
FIG. 6 is a view suitable for explaining curving deformation of the catheter when an operating force in a pushing direction is exerted on the control wire in the catheter shown in FIG. 1.
FIG. 7 is a vertical cross sectional view showing a principal part of a catheter according to another practical embodiment of the first practical embodiment of the present invention, corresponding to FIG. 2.
FIG. 8 is a cross sectional view taken along line 8-8 of FIG. 7.
FIG. 9 is a vertical cross sectional view schematically showing a principal part of a catheter according to a second practical embodiment of the present invention.
FIG. 10 is a view suitable for explaining curving deformation of the catheter when an operating force in a pulling direction is exerted on a control wire in the catheter shown in FIG. 9.
FIG. 11 is a view suitable for explaining curving deformation of the catheter when an operating force in a pushing direction is exerted on the control wire in the catheter shown in FIG. 9.
FIG. 12 is a plan view schematically showing a principal part of a catheter according to a third practical embodiment of the present invention.
FIG. 13 is a cross sectional view taken along line 13-13 of FIG. 12.
FIG. 14 is a cross sectional view taken along line 14-14 of FIG. 12.
FIG. 15 is a vertical cross sectional view schematically showing a principal part of a catheter according to a fourth practical embodiment of the present invention.
FIG. 16 is a view suitable for explaining curving deformation of the catheter when an operating force in a pulling direction is exerted on a control wire in the catheter shown in FIG. 15.
FIG. 17 is a vertical cross sectional view schematically showing a principal part of a catheter according to a fifth practical embodiment of the present invention.
FIG. 18 is a view suitable for explaining curving deformation of the catheter when an operating force in a pulling direction is exerted on a control wire in the catheter shown in FIG. 17.
FIG. 19 is a vertical cross sectional view schematically showing a principal part of a catheter according to a sixth practical embodiment of the present invention.
FIG. 20 is a view suitable for explaining curving deformation of the catheter when an operating force in a pulling direction is exerted on a control wire in the catheter shown in FIG. 19.
FIG. 21 is a vertical cross sectional view schematically showing a principal part of a catheter according to a seventh practical embodiment of the present invention.
FIG. 22 is a view suitable for explaining curving deformation of the catheter when an operating force in a pulling direction is exerted on a control wire in the catheter shown in in FIG. 21.
FIGS. 23A-23D are transverse cross sectional views each showing a catheter according to another practical embodiment of the present invention, corresponding to FIG. 4.
FIG. 24 is a transverse cross sectional view showing a catheter according to yet another practical embodiment of the present invention, corresponding to FIG. 4.
FIGS. 25A and 25B are transverse cross sectional views each showing a catheter according to yet another practical embodiment of the present invention, corresponding to FIG. 4.
FIG. 26 is a transverse cross sectional view showing a catheter according to yet another practical embodiment of the present invention, corresponding to FIG. 4.
FIGS. 27A-27C are transverse cross sectional views each showing a catheter according to yet another practical embodiment of the present invention, corresponding to FIG. 3.
FIG. 28 is a transverse cross sectional view showing a catheter as another practical embodiment of the third practical embodiment of the present invention, corresponding to FIG. 14.

### EMBODIMENTS FOR CARRYING OUT THE INVENTION

Hereinafter, practical embodiments of the present invention will be described in reference to the drawings.

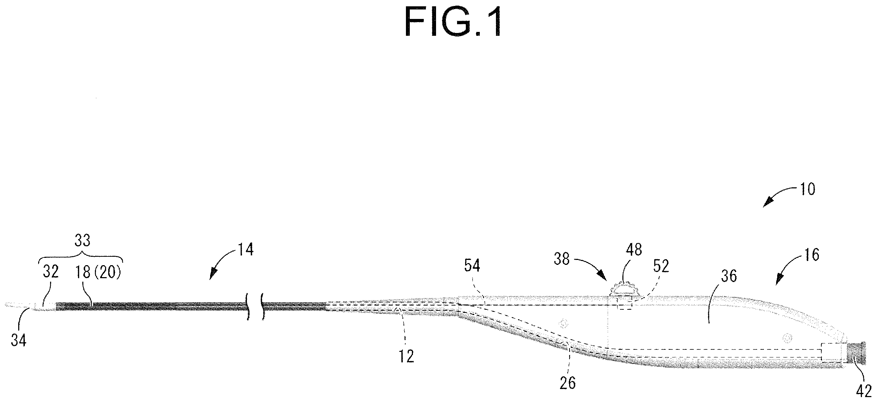

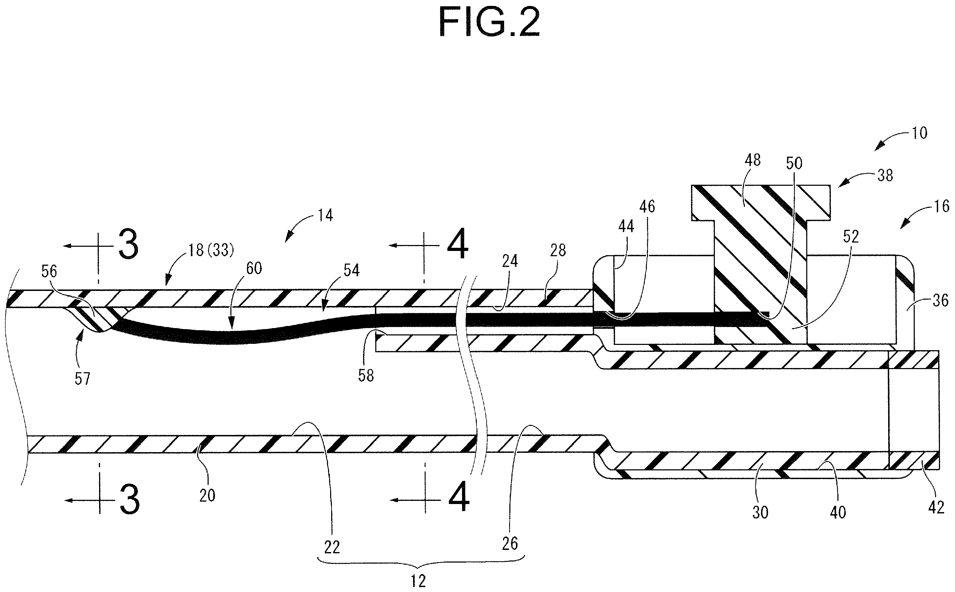

First, in FIGS. 1 to 4, there is depicted a suction catheter 10 used for a lower-limb blood vessel as a first practical embodiment of a catheter according to the present invention. The suction catheter 10 includes in its inside a suction lumen 12 serving as a main lumen which opens to the distal end and the proximal end while extending across the entire length in the lengthwise direction. When the suction catheter 10 is inserted into the blood vessel of the lower limb and the negative pressure source is connected to the proximal end side of the suction lumen 12, the thrombus and the like in the blood vessel of the lower limb is suctioned through the suction lumen 12. In the following description, the lengthwise direction refers to the left-right direction in FIG. 1. In addition, the distal end side refers to the left side in FIG. 1, while the proximal end side refers to the right side in FIG. 1. Furthermore, "upward" refers to upward in FIG. 1, while "downward" refers to downward in FIG. 1. Note that the vertical cross sectional view and the transverse cross sectional views of the suction catheter 10 shown in FIGS. 2 to 4 schematically show the suction catheter 10, and do not show exactly the shape and size of the suction catheter 10.

More specifically, the suction catheter 10 includes a catheter main body 14 and a controller 16 provided on the proximal end side of the catheter main body 14.

The catheter main body 14 includes an elongated, tubular main body shaft 18 having a peripheral wall 20 extending in the lengthwise direction. The main body shaft 18 is formed of a soft synthetic resin, and for example, polyamide, polyamide elastomer, polyvinyl chloride, polyurethane, polyimide, polyethylene, polyethylene elastomer, polypropylene, polytetrafluoroethylene, polyetherketone, polyvinylidene fluoride or the like may be adopted. Since the suction catheter 10 of the present practical embodiment is used for lower-limb blood vessels, its outside diameter dimension is larger than that of, for example, a cardiovascular catheter, and the outside diameter dimension of the main body shaft 18 is about 2 mm. Here, the lower-limb blood vessel includes, for example, iliac artery and vein, femoral artery and vein, popliteal (below-knee) artery and vein, and the like.

As shown in FIGS. 2 to 4, the main body shaft 18 has a single lumen structure at the distal end portion and the proximal end portion, while at the axially middle portion, having a double lumen structure in which two lumens are arranged in parallel. That is, in the distal end portion of the main body shaft 18, a central lumen 22 extending in the lengthwise direction is formed on the radially inner side of the peripheral wall 20, while in the axially middle portion, a first lumen 24 and a second lumen 26 extending in the lengthwise direction are formed. The second lumen 26 extends to the proximal end portion of the main body shaft 18. The portion that is formed as a single lumen structure of the central lumen 22 and the portion that is formed as a double lumen structure of the first and second lumens 24, 26 may be formed separately and secured later to each other. However, in the present practical embodiment, these are integrally formed.

The first lumen 24 has a smaller diameter than those of the central lumen 22 and the second lumen 26, and the first lumen 24 and the second lumen 26 are arranged in parallel in the vertical direction. That is, a part of a peripheral wall 28 of the first lumen 24 is constituted by the peripheral wall 20 of the main body shaft 18, and the other part on the circumference is constituted in common with a peripheral wall 30 of the second lumen 26.

The central lumen 22 and the second lumen 26 constitute the suction lumen 12 serving as a main lumen extending through the main body shaft 18 across the entire length thereof in the lengthwise direction.

Further, a distal end tip 32 is secured to the distal end of the main body shaft 18. The distal end tip 32 has a roughly tubular shape overall, and the suction lumen 12 and the inner hole of the distal end tip 32 communicate with each other. The main body shaft 18 and the distal end tip 32 constitute an elongated, tubular shaft member 33 extending in the lengthwise direction of the catheter main body 14. In the present practical embodiment, the distal end tip 32, which is formed separately from the main body shaft 18, is secured later to the main body shaft 18. However, for example, the distal end tip may be integrally formed with the main body shaft. A notch-shaped opening part 34 is formed in a part of the peripheral wall of the distal end tip 32 (the lower side in FIG. 1), and the distal end of the suction lumen 12 opens to the outside via the opening part 34.

In the present practical embodiment, a guidewire lumen through which the guidewire is inserted is constituted by the inner hole of the distal end tip 32 and the suction lumen 12, and the guidewire lumen extends across roughly the entire length of the suction catheter 10. Therefore, the suction catheter 10 of the present practical embodiment is an over-the-wire type catheter.

Meanwhile, the controller 16 includes a body 36 having a generally rectangular parallelepiped shape overall and an operating part 38 that is displaceable in the lengthwise direction with respect to the body 36. The body 36 is shaped and sized so that the user can operate the operating part 38 with his or her thumb while grasping it with one hand. In order to facilitate grasping by a user, the body 36 of the present practical embodiment is configured such that at the distal end portion, the lower face gradually slopes upward toward the distal end side, while at the proximal end portion, the upper face gradually slopes downward toward the proximal end side.

A through hole 40 penetrating in the lengthwise direction is formed below the body 36, and a tubular connector 42 is inserted and fixed to the proximal end side opening of the through hole 40. The proximal end portion of the main body shaft 18 (the peripheral wall 30 constituting the second lumen 26) is inserted through the through hole 40 of the body 36, and the proximal end of the peripheral wall 28 constituting the first lumen 24 is secured to the distal end of the body 36, so that the controller 16 is attached to the catheter main body 14. The suction lumen 12 (the second lumen 26) and the inner hole of the connector 42 communicate with each other. That is, the proximal end of the suction lumen 12 opens to the outside via the connector 42.

Further, on the upper face of the body 36, a slit 44 extending in the lengthwise direction with a predetermined dimension is formed. The slit 44 opens upward. Besides, an insertion hole 46 penetrating in the lengthwise direction is formed in the distal end portion of the body 36, and the internal space at the lower portion of the slit 44 mutually communicates with the first lumen 24 through the insertion hole 46.

Meanwhile, the operating part 38 is generally rectangular parallelepiped overall with a slightly smaller width dimension than that of the slit 44, and is fitted in the slit 44. Further, the upper portion of the operating part 38 protrudes from the body 36, and the user can operate the protruding portion of the operating part 38 from the body 36 with a thumb or the like while grasping the body 36. Therefore, the protruding portion of the operating part 38 from the body 36 serves as an input part 48 for substantially operating the operating part 38.

On the other hand, in the lower portion of the operating part 38, an insertion hole 50 opening to the distal end side is provided, and a proximal end of a control wire 54, which will be described later, is inserted and secured to the insertion hole 50 by being subjected to processing such as bonding as necessary. Therefore, the lower portion of the operating part 38 serves as a wire holding portion 52 for holding the control wire 54.

Here, a control wire 54 for controlling the curving deformation of the suction catheter 10 is inserted through the first lumen 24. The control wire 54 is constituted by, for example, a narrow metal or a linear body of resin and has flexibility, while being made of stainless steel, Ni-Ti alloy, nylon, polyethylene, polyester, polypropylene, carbon fiber or the like. Therefore, the first lumen 24 serves as a control lumen through which the control wire 54 is inserted.

The control wire 54 extends inside the first lumen 24, and extends into the internal space of the slit 44 through the insertion hole 46 provided at the distal end of the body 36. The proximal end of the control wire 54 is inserted into the insertion hole 50 of the operating part 38 and secured to the wire holding portion 52.

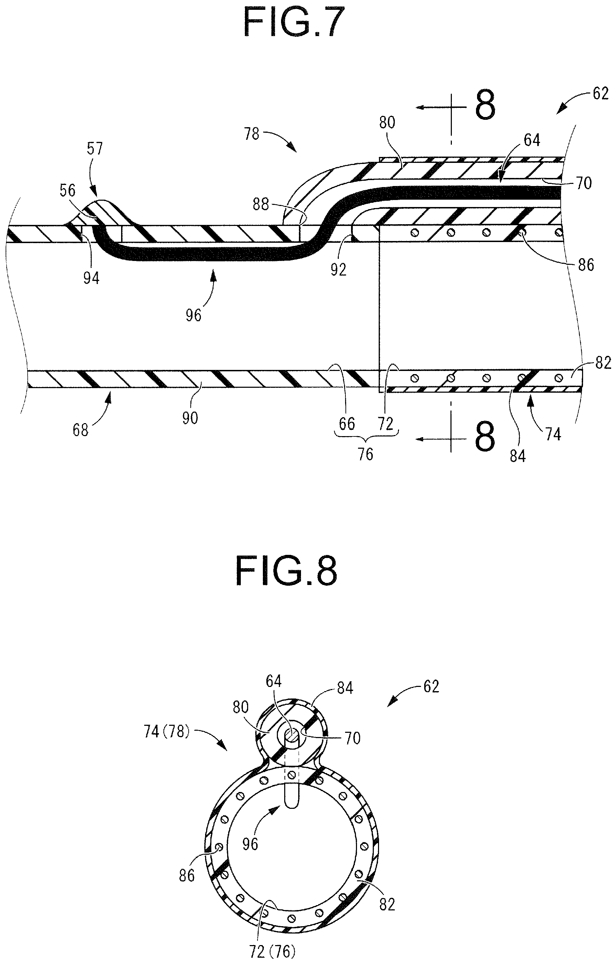

Meanwhile, the distal end of the control wire 54 extends further to the distal end side than the first lumen 24, and is fixed to the upper inner surface of the central lumen 22 (the main body shaft 18) by bonding with an adhesive 56 or welding. The fixed position of the distal end of the control wire 54 on the main body shaft 18 serves as a fixing part 57. That is, the distal end portion of the control wire 54 protrudes from the first lumen (the control lumen) 24 through a distal end opening 58 of the first lumen 24 serving as the window part, and the distal end opening (the window part) 58 of the first lumen 24 is provided further on the proximal end side than the fixed position (the fixing part 57) of the control wire 54 to the main body shaft 18. In addition, the control wire 54, which has entered the inside of the central lumen 22 as shown in FIG. 7 described later, may protrude to the outside through another through hole and be secured to the radially outer surface of the main body shaft 18.

It is preferable that these fixing part 57 and the window part 58 are provided at positions away from each other by 10 to 50 mm in the lengthwise direction, and more preferably, they are provided at positions away from each other by 20 to 40 mm. That is, if the remote distance between the fixing part 57 and the window part 58 is smaller than 10 mm, during deformation of the suction catheter 10 which will be described later, the amount of deformation in the direction orthogonal to the lengthwise direction is too small. Thus, the opening part 34 at the distal end of the suction catheter 10 cannot be brought sufficiently close to the inner wall of the blood vessel, and there is a risk that a thrombus or the like adhering to the inner wall of the blood vessel may not be sufficiently suctioned. In addition, if the remote distance between the fixing part 57 and the window part 58 is greater than 50 mm, the starting point of bending in the suction catheter 10 is too far from the distal end of the suction catheter 10. Thus, the curvature of the bent portion of the suction catheter 10 is too small with respect to the curvature of the blood vessels of the lower limb (for example, an external iliac artery and a superficial femoral artery), and there is a risk that it may be difficult to deform the suction catheter into a shape corresponding to the curved shape of the blood vessel. Moreover, the amount of operation of the control wire 54, that is, the amount of operation load exerted on the control wire 54, becomes too large, posing a risk of increasing stress applied to the blood vessel.

Besides, it is preferable that the remote distance from the distal end of the suction catheter 10 (the shaft member 33) to the fixing part 57 is set within the range of 10 to 40 mm in the lengthwise direction, and more preferably, the distance is set within the range of 20 to 30 mm. That is, if the remote distance between the distal end of the suction catheter 10 and the fixing part 57 is smaller than 10 mm, during deformation of the suction catheter 10 which will be described later, the amount of deformation in the direction orthogonal to the lengthwise direction is too small. Thus, the opening part 34 at the distal end of the suction catheter 10 cannot be brought sufficiently close to the inner wall of the blood vessel, and there is a risk that a thrombus or the like adhering to the inner wall of the blood vessel may not be sufficiently suctioned. In addition, if the remote distance between the distal end of the suction catheter 10 and the fixing part 57 is greater than 40 mm, the starting point of bending in the suction catheter 10 is too far from the distal end of the suction catheter 10. Thus, the curvature of the bent portion of the suction catheter 10 is too small with respect to the curvature of the blood vessels of the lower limb (for example, an external iliac artery and a superficial femoral artery), and there is a risk that it may be difficult to deform the suction catheter into a shape corresponding to the curved shape of the blood vessel. Moreover, the amount of operation of the control wire 54, that is, the amount of operation load exerted on the control wire 54, becomes too large, posing a risk of increasing stress applied to the blood vessel.

Then, the control wire 54 is, at its protruding portion from the distal end opening 58 of the first lumen 24, exposed to the inside of the suction lumen (main lumen) 12 serving as a separate lumen which is a separate internal space from the first lumen 24. An exposed part 60 of the control wire 54 has a predetermined length dimension, and in the present practical embodiment, the length dimension is made equal to the remote distance between the fixing part 57 and the window part 58 in the lengthwise direction.

Although the cross-sectional shape of the control wire 54 is not limited in any way, the control wire 54 of the present practical embodiment extends across roughly the entire length in the lengthwise direction with a circular constant cross-sectional shape.

The distal end side of the suction catheter 10 having such a structure is inserted into the blood vessel, while a Y-shaped connector is connected in an internally arranged state to the connector 42 provided on the proximal end side. Then, by connecting a negative pressure source to the Y-shaped connector, blood including a thrombus and the like is suctioned through the suction lumen 12. It should be appreciated that such a Y-shaped connector may be connected to the connector 42 in an externally fitted state.

Here, with the suction catheter 10, by operating the operating part 38 of the controller 16 from the outside and displacing the operating part 38 within the slit 44 in the lengthwise direction, it is possible to exert an operating force in the lengthwise direction on the control wire 54 fixed to the operating part 38. This enables the distal end portion of the suction catheter 10 to deform to curve.

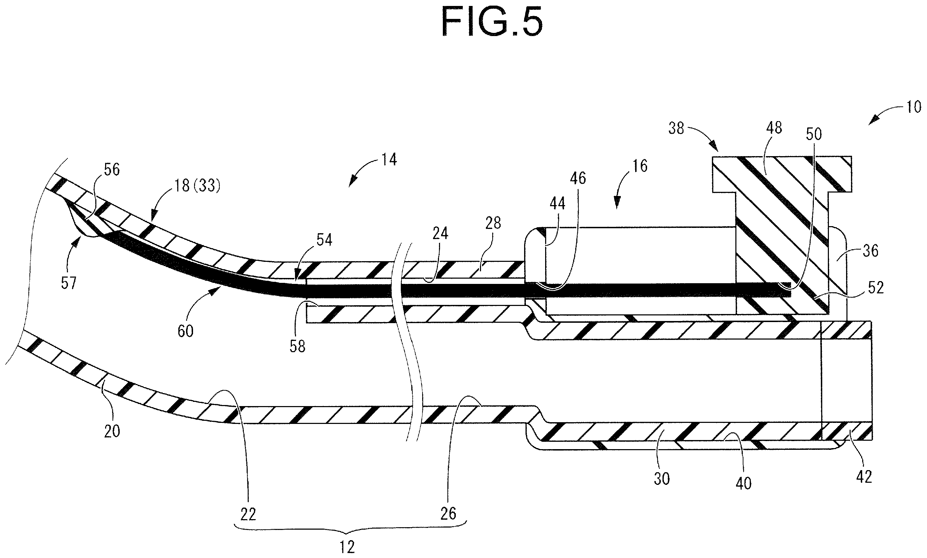

That is, as shown in FIG. 5, by displacing the operating part 38 to the proximal end side with respect to the body 36, an operating force in the pulling direction is exerted on the control wire 54. Accordingly, the peripheral wall 20 of the portion to which the distal end of the control wire 54 is fixed is pulled toward the proximal end side. Therefore, in the peripheral wall 20 of the main body shaft 18, the upper wall part in the drawing is compressed in the lengthwise direction, and the length dimension is made smaller than that of the lower wall part, so that the distal end of the suction catheter 10 is deformed to curve upward.

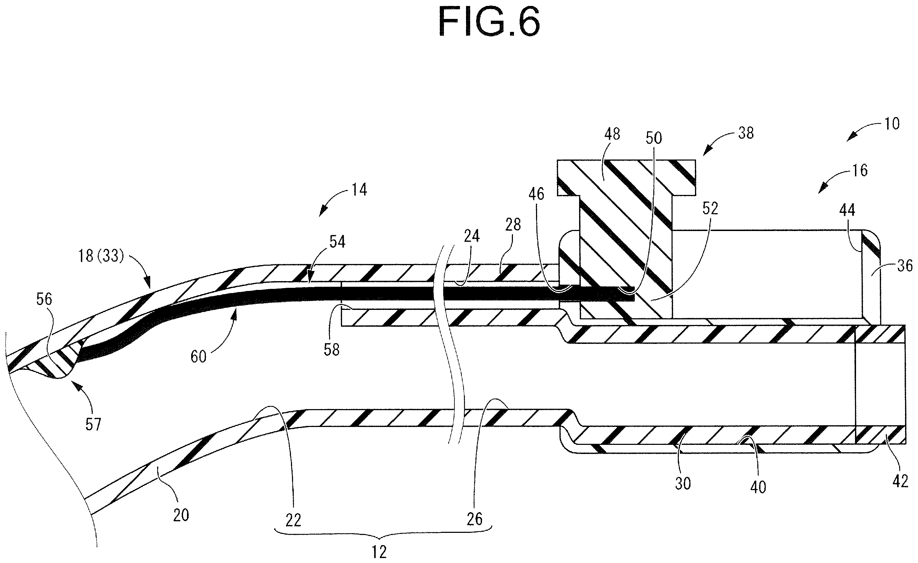

On the other hand, as shown in FIG. 6, by displacing the operating part 38 to the distal end side with respect to the body 36, an operating force in the pushing direction is exerted on the control wire 54. Accordingly, the peripheral wall 20 of the portion to which the distal end of the control wire 54 is fixed is pushed toward the distal end side. Therefore, in the peripheral wall 20 of the main body shaft 18, the upper wall part in the drawing is pulled in the lengthwise direction, and the length dimension is made larger than that of the lower wall part, so that the distal end of the suction catheter 10 is deformed to curve downward.

With the suction catheter 10 having the structure as described above, by deforming its distal end portion to curve, it is possible to bring the opening part 34 close to the wall surface of the blood vessel. This may improve the suction efficiency of the thrombus adhering to the inner wall of the blood vessel, for example.

Here, since the control wire 54 is exposed through the window part (the distal end opening 58 of the first lumen 24) into the main lumen (the suction lumen 12), a large protruding deformation of the control wire 54 into the main lumen is permitted. This will reduce increase in the sliding contact resistance caused by the contact of the first lumen 24 with the peripheral wall 28, while preventing inhibition of deformation of the suction catheter 10 due to deformation restraint of the control wire 54.

FIGS. 7 and 8 depict principal parts of a suction catheter 62 used for a lower-limb blood vessel, which is another embodiment of the suction catheter 10 according to the first practical embodiment. In this practical embodiment, since the same structure as in the first practical embodiment can be adopted for the distal end portion and the proximal end portion of the suction catheter 62, in FIG. 7, only the distal end portion of a control wire 64 is shown.

In the first practical embodiment, in the main body shaft 18, the portion that is formed as a single lumen structure of the central lumen 22 and the portion that is formed as a double lumen structure of the first and second lumens 24, 26 are integrally formed. However, in the present embodiment, they are separately formed and secured later to each other. Specifically, in the present embodiment, a proximal end tube 74 including a first lumen 70 and a second lumen 72 is secured to a distal end tube 68 including a central lumen 66 by bonding, welding or the like. The central lumen 66 and the second lumen 72 communicate with each other, and these lumens constitute a suction lumen 76 serving as a main lumen. Further, in the present embodiment, a shaft member 78 is constituted by including the distal end tube 68 and the proximal end tube 74.

In the first practical embodiment, the peripheral wall 28 of the first lumen 24 and the peripheral wall 30 of the second lumen 26 are integrally formed, and these walls are constituted in common in part. However, in the present embodiment, a peripheral wall 80 of the first lumen 70 and a peripheral wall 82 of the second lumen 72 are formed separately and independent of each other. The peripheral walls 80, 82 are overlapped in the vertical direction and covered with a cover tube 84, and then the peripheral walls 80, 82 are intimately fixed by thermal shrinkage, whereby the proximal end tube 74 is constituted. The peripheral wall 82 of the second lumen 72 is reinforced by a braided wire 86 such as a metal being embedded therein.

The control wire 64 is inserted through the first lumen 70 serving as a control lumen, and the control wire 64 protruding from a distal end opening 88 of the first lumen 70 serving as a window part enters the inside of the central lumen 66 through a through hole 92 provided in a peripheral wall 90 of the distal end tube 68. Besides, the control wire 64 that has entered the inside of the central lumen 66 protrudes to the outside through another through hole 94 and is secured to the radially outer surface of the distal end tube 68 with an adhesive 56 or the like. The distal end opening 88 of the first lumen 70 is secured to the radially outer side opening edge of the through hole 92 provided in the peripheral wall 90 of the distal end tube 68, so that the first lumen 70 and the central lumen 66 are in communication with no gap. The adhesive 56 provided on the radially outer surface of the distal end tube 68 closes the through hole 94 provided in the peripheral wall 90 from the radially outer side. Accordingly, the control wire 64 is configured so as not to be exposed to the outside of the suction catheter 62.

In the suction catheter 62 having the above-described structure as well, the control wire 64 protruding from the window part (the distal end opening) 88 of the control lumen (the first lumen) 70 is provided with a exposed part 96 exposed to the main lumen serving as a separate lumen (the suction lumen) 76, which is a separate internal space. Thus, the same effect as in the first practical embodiment can be exhibited.

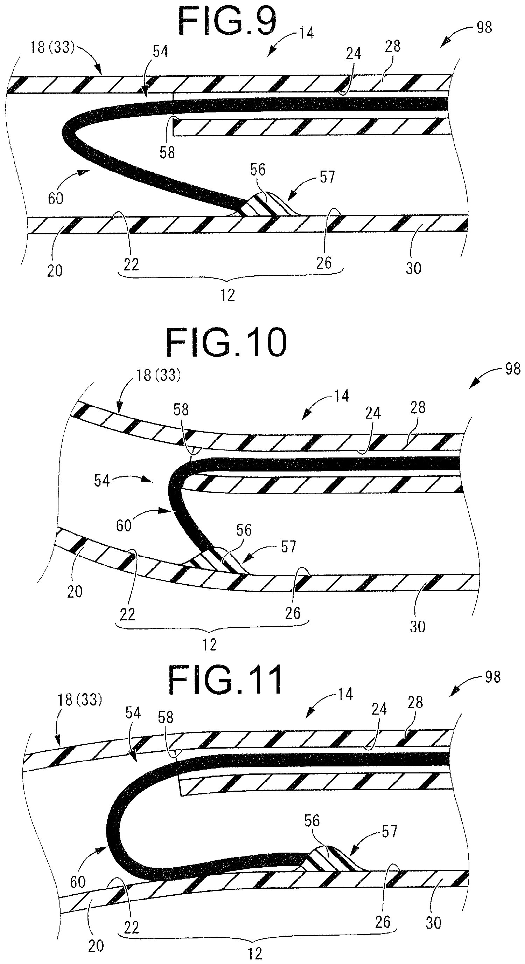

Next, FIG. 9 depicts a suction catheter 98 used for a lower-limb blood vessel as a second practical embodiment of the catheter according to the present invention. Since the structure of the suction catheter 98 other than the distal end portion of the control wire 54 is the same as that of the preceding first practical embodiment, illustration of the portion other than the distal end portion of the control wire 54 is omitted in FIGS. 9 to 11. In the following description, components and parts that are substantially identical with those in the preceding first practical embodiment will be assigned like symbols and not described in any detail.

In the suction catheter 98 of the present practical embodiment, the control wire 54 protruding from the distal end portion of the first lumen (the control lumen) 24 makes a U-turn toward the proximal end side at the distal end side of the distal end opening (the window part) 58, and is fixed to the lower inner surface of the second lumen 26 with the adhesive 56. That is, the distal end opening 58 of the first lumen 24 is provided further on the distal end side than the fixed position of the control wire 54 to the main body shaft 18 (the fixing part 57).

With the suction catheter 98 having the above-described structure as well, by operating the operating part (38) of the controller (16), the distal end portion is deformed to curve. Specifically, as shown in FIG. 10, by pulling the operating part (38) toward the proximal end side, an operating force in the pulling direction is exerted on the control wire 54. Accordingly, in the peripheral wall 20 of the main body shaft 18, the lower wall part is pulled toward the distal end side, and the length dimension of the lower wall part is made larger than that of the upper wall part, so that the distal end portion of the suction catheter 98 is deformed to curve upward. In the present practical embodiment in particular, by applying the operating force in the pulling direction to the control wire 54, stress is exerted on the opening edge of the distal end opening (the window part) 58 of the first lumen 24, so that the peripheral wall 20 of the main body shaft 18 is deformed to curve together with the distal end of the peripheral wall 28 of the first lumen 24.

On the other hand, as shown in FIG. 11, by pushing the operating part (38) toward the distal end side, an operating force in the pushing direction is exerted on the control wire 54. Accordingly, in the peripheral wall 20 of the main body shaft 18, the lower wall part is pushed toward the proximal end side, and the length dimension of the lower wall part is made smaller than that of the upper wall part, so that the distal end portion of the suction catheter 98 is deformed to curve downward. In addition, in the present practical embodiment, by the control wire 54 being pushed toward the distal end side, the control wire 54 comes into contact with the lower part of the peripheral wall 20 of the main body shaft 18, and the distal end portion of the suction catheter 98 is deformed to curve downward by the pressing force of the control wire 54 as well.

In the suction catheter 98 of the present practical embodiment as well, the distal end portion of the control wire 54 is exposed to the suction lumen (the main lumen) 12 serving as a separate lumen, which is a separate internal space. Thus, the same effect as in the first practical embodiment can be exhibited.

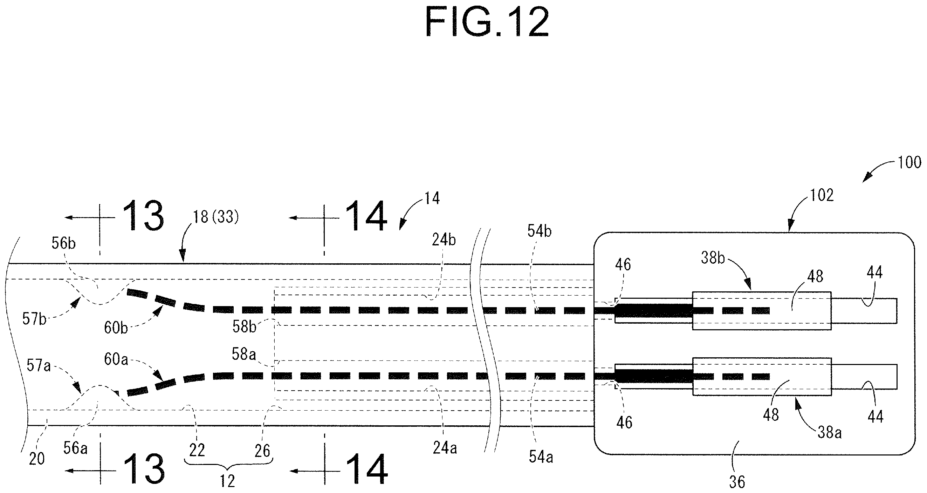

Next, FIGS. 12 to 14 depict a suction catheter 100 used for a lower-limb blood vessel as a third practical embodiment of the catheter according to the present invention. The suction catheter 100 of the present practical embodiment is provided with two control wires 54a, 54b, which are independent of each other. Specifically, a controller 102 of the present practical embodiment is provided with two operating part 38a, 38b, and one operating part 38a (38b) is fixed to the proximal end of one control wire 54a (54b).

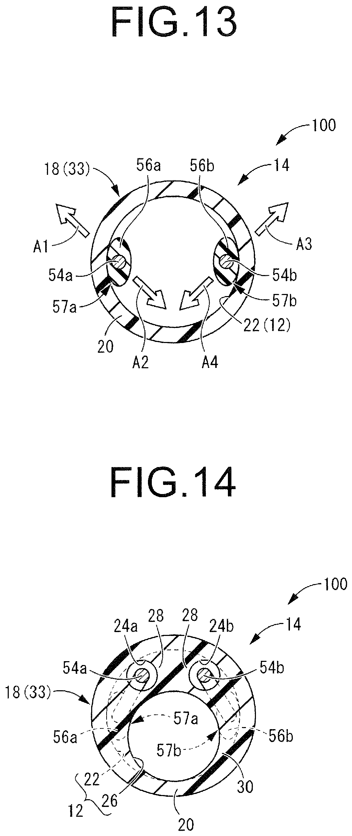

Further, the distal ends of the control wires 54a, 54b are fixed to the inner surface of the peripheral wall 20 of the main body shaft 18 at different positions from each other in the circumferential direction (in the present practical embodiment, both sides in the left-right direction in FIG. 13) by adhesives 56a, 56b.

Moreover, in the present practical embodiment, inside the main body shaft 18, two first lumens 24a, 24b are provided correspondingly to the control wires 54a, 54b, while a single second lumen 26 is provided. The two first lumens 24a, 24b are arranged in parallel in the left-right direction in FIG. 13, and the second lumen 26 is provided below the first lumens 24a, 24b. Then, one control wire 54a (54b) is inserted through one first lumen 24a (24b).

In the present practical embodiment, as shown in FIG. 14, the first lumens 24a, 24b and the control wires 54a, 54b are provided so as to be roughly symmetrical in the left-right direction with respect to the diametrical line extending in the vertical direction in the drawing, and fixing parts 57a, 57b of the control wires 54a, 54b formed by the adhesives 56a and 56b are provided in the same way. Specifically, the first lumen 24a extending in the axial direction is formed on the left side of the diametrical line, and the fixing part 57a of the control wire 54a formed by the adhesive 56a is provided at the position spaced apart from the first lumen 24a by a predetermined distance counterclockwise in the circumferential direction. Besides, the first lumen 24b extending in the axial direction is formed on the right side of the diametrical line, and the fixing part 57b of the control wire 54b formed by the adhesive 56b is provided at the position spaced apart from the first lumen 24b by a predetermined distance clockwise in the circumferential direction.

It should be appreciated that distal end openings 58a, 58b of the first lumens 24a, 24b are away from the fixing parts 57a, 57b of the control wires 54a, 54b formed by the adhesives 56a, 56b toward the proximal end side by a predetermined distance in the axial direction. That is, between the distal end openings 58a, 58b serving as the window parts of the first lumens 24a, 24b and the adhesives 56a, 56b (the fixing parts 57a, 57b), the control wires 54a, 54b are provided with exposed parts 60a, 60b exposed to the suction lumen 12.

In the present practical embodiment in particular, the pair of first lumens 24a, 24b are formed close to each other on both sides sandwiching the diametrical line, while the fixing parts 57a, 57b to the pair of control wires 54a, 54b formed by the adhesives 56a, 56b are positioned on the diametrical line extending in the left-right direction in FIG. 14. With this arrangement, as will be described later, the operating force exerted through each of the control wires 54a, 54b is configured to be applied to the peripheral wall of the suction catheter 100 as an external force to the direction in which each of the control wires 54a, 54b disposed astride the fixing parts 57a, 57b formed by the respective adhesives 56a, 56b and the distal end openings 58a, 58b of the first lumens 24a, 24b extends, or as a resultant force exerted on each of the adhesives 56a, 56b (the fixing parts 57a, 57b) and each of the distal end openings 58a, 58b of the first lumens 24a, 24b.

In the suction catheter 100 of the present practical embodiment having the above-described structure as well, portions of the control wires 54a, 54b protruding from the distal end openings (window parts) 58a, 58b of the first lumens 24a, 24b serve as the exposed parts 60a, 60b exposed to the suction lumen 12 serving as a separate lumen, which is a separate internal space.

In the suction catheter 100 as described above, when the operating part 38a is operated to exert an operating force in the pulling direction on the left side control wire 54a in FIG. 13, the distal end portion of the suction catheter 100 is deformed to curve roughly in the direction of a white arrow A1 in FIG. 13 (the upper left direction in FIG. 13), while in the case of exerting an operating force in the pushing direction, the distal end portion thereof is deformed to curve roughly in the direction of a white arrow A2 in FIG. 13 (the lower right direction in FIG. 13). On the other hand, when the operating part 38b is operated to exert an operating force in the pulling direction on the right side control wire 54b in FIG. 13, the distal end portion of the suction catheter 100 is deformed to curve roughly in the direction of a white arrow A3 in FIG. 13 (the upper right direction in FIG. 13), while in the case of exerting an operating force in the pushing direction, the distal end portion thereof is deformed to curve roughly in the direction of a white arrow A4 in FIG. 13 (the lower left direction in FIG. 13).

In the suction catheter 100 of the present practical embodiment having the above-described structure as well, the exposed parts 60a, 60b exposed to the suction lumen 12 are provided at the distal end portions of the control wires 54a, 54b. Thus, the same effect as in the first practical embodiment can be exhibited.

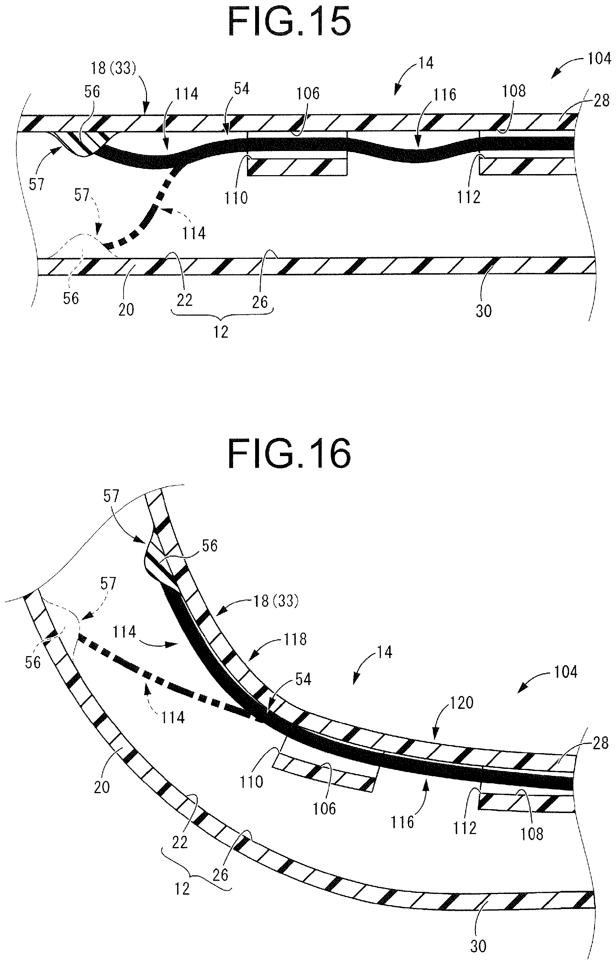

Next, FIG. 15 depicts a suction catheter 104 used for a lower-limb blood vessel as a fourth practical embodiment of the catheter according to the present invention. In the present practical embodiment as well, the illustration of the portion other than the distal end portion of the control wire 54 is omitted.

Specifically, the present practical embodiment has a structure in which a plurality of window parts are provided in the lengthwise direction of the first lumen. In the present practical embodiment in particular, each window part is formed to have a size across nearly the entire circumference of the peripheral wall of the first lumen, so that it can also be assumed that substantially a plurality of first lumens divided in the lengthwise direction by the respective window parts. According to this viewpoint, inside the main body shaft 18 of the present practical embodiment, there are provided a distal end first lumen 106 and a proximal end first lumen 108 serving as control lumens. These distal end and proximal end first lumens 106, 108 are spaced away from each other in the lengthwise direction, and the respective distal end openings comprise first and second distal end openings 110, 112 serving as window parts.

The control wire 54 is inserted through each of the distal end and proximal end first lumens 106, 108, and is fixed to the upper side of the inner surface of the main body shaft 18 by the adhesive 56 further on the distal end side than the distal end first lumen 106. It should be appreciated that the distal end of the control wire 54 may be fixed at any position on the circumference of the inner surface of the main body shaft 18. For example, an embodiment in which the fixed part (the fixing part 57) of the distal end of the control wire 54 is set to the lower side situated in opposition to the distal end first lumen 106 in the diametrical direction is indicated by the chain double-dashed line in FIG. 15.

Specifically, in the control wire 54, a portion which protrudes from the first distal end opening 110 so as to be exposed to the suction lumen (main lumen) 12 serves as a first exposed part 114, and a portion which protrudes from the second distal end opening 112 so as to be exposed to the second lumen 26 serves as a second exposed part 116.

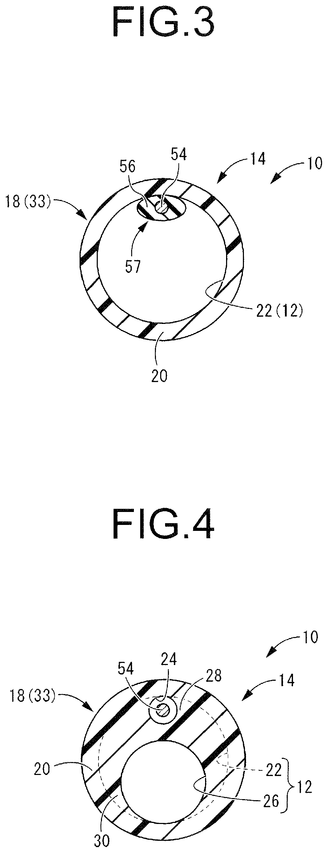

In the suction catheter 104 having such a structure, when the operating part (38) is displaced to the proximal end side and an operating force in the pulling direction is exerted on the control wire 54, as shown in FIG. 16, the distal end portion of the suction catheter 104 is deformed to curve upward. In particular, the transverse cross sectional area of the main body shaft 18 is made different between the portion where the distal end and proximal end first lumens 106, 108 are formed and the portion where they are not formed (see FIGS. 3 and 4), and deformation rigidity of the portion where the distal end and proximal end first lumens 106, 108 are not formed is made small. Therefore, when the suction catheter 104 is deformed to curve, the portion where the distal end and proximal end first lumens 106, 108 are not formed readily curves. Specifically, although it is hard to see in the drawing, in the main body shaft 18 during the curving deformation, the portion further on the distal end side than the distal end first lumen 106 comprises a first curved portion 118 having a large curvature, and the portion between the distal end first lumen 106 and the proximal end first lumen 108 comprises a second curved portion 120 having a large curvature, making it possible to have a multistep curved shape curved in two steps at two bending points spaced apart by a predetermined distance in the lengthwise direction. In particular, when an operating force in the pulling direction is exerted on the control wire 54, the stress exerted on the edge part of the window part 110 of the distal end first lumen 106 is greater than the stress exerted on the edge part of the window part 112 of the proximal end first lumen 108. Thus, the curvature of the first curved portion 118 is configured to be larger than that of the second curved portion 120.

Meanwhile, when the operating part (38) is displaced to the distal end side and an operating force in the pushing direction is exerted on the control wire 54, the suction catheter 104 deforms to curve in the same way as in the first practical embodiment, therefore illustration is omitted. Besides, the embodiment of deformation of the control wire 54 in the case where the distal end of the control wire 54 is fixed to the lower side of the main body shaft 18 is as indicated by the chain double-dashed line in FIG. 16. Specifically, when a pulling force is exerted on the control wire 54, the deforming force acts in the direction in which the length of the control wire 54 disposed across the first distal end opening 110 of the distal end first lumen 106 and the fixing part 57 on the diametrically opposite side is shortened, as shown in FIG. 16, the embodiment of deformation of the suction catheter 104 is the same as in the case where the distal end of the control wire 54 is fixed to the upper side of the main body shaft 18. It is also possible to control the direction of bending applied to the suction catheter 104 by adjusting and changing the position of the fixing part 57 of the distal end of the control wire 54 in the circumferential direction.

In the suction catheter 104 of the present practical embodiment as well, the control wire 54 is provided with the first and second exposed parts 114, 116 exposed to the suction lumen 12. Thus, the same effect as in the first practical embodiment can be exhibited.

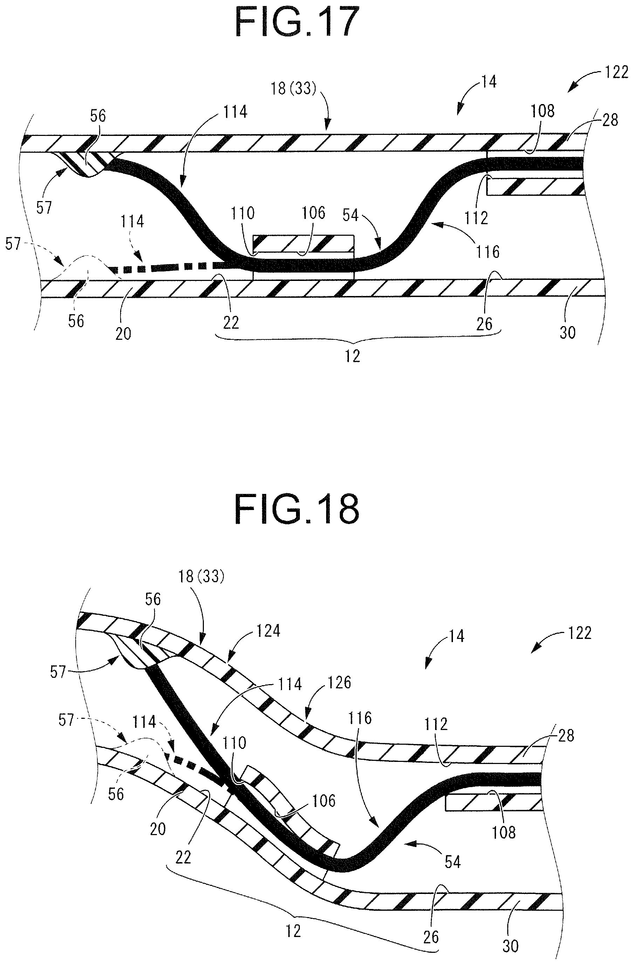

Next, FIG. 17 depicts a suction catheter 122 used for a lower-limb blood vessel as a fifth practical embodiment of the catheter according to the present invention. In the present practical embodiment, in the same way as in the fourth practical embodiment, a plurality of first lumens are provided, and the circumferential positions of these first lumens are made different from each other. In the present practical embodiment as well, the illustration of the portion other than the distal end portion of the control wire 54 is omitted.

That is, in the present practical embodiment, the distal end first lumen 106 and the proximal end first lumen 108 are provided at different positions in the lengthwise direction, while being provided at different positions in the circumferential direction (in the present practical embodiment, each side in the vertical direction). Specifically, the distal end first lumen 106 is provided on the lower side of the main body shaft 18, while the proximal end first lumen 108 is provided on the upper side of the main body shaft 18. In short, a part of the peripheral wall of the distal end first lumen 106 is constituted by the lower portion of the peripheral wall 20 of the main body shaft 18, while a part of the peripheral wall of the proximal end first lumen 108 is constituted by the upper portion of the peripheral wall 20 of the main body shaft 18.

The control wire 54 is inserted through the distal end and proximal end first lumens 106, 108, and the distal end of the control wire 54 is fixed to the upper side of the inner surface of the main body shaft 18 with the adhesive 56 further on the distal end side than the distal end first lumen 106. An embodiment in which the distal end of the control wire 54 is fixed to the lower side of the inner surface of the main body shaft 18 is indicated by the chain double-dashed line in FIG. 17.

In the present practical embodiment as well, in the control wire 54, a portion protruding from the first distal end opening 110, which is the distal end opening of the distal end first lumen 106, and a portion protruding from the second distal end opening 112, which is the distal end opening of the proximal end first lumen 108, are exposed to the suction lumen 12.

In the suction catheter 122 having the above-described structure as well, as shown in FIG. 18, by displacing the operating part (38) to the proximal end side, an operating force in the pulling direction is exerted on the control wire 54, and the distal end portion of the suction catheter 122 curves upward. At this time, in the suction catheter 122, the deformation rigidity in the radially outer portion of the first exposed part 114 is made relatively small, while the deformation rigidity in the radially outer portion of the distal end first lumen 106 is made relatively large. Therefore, in the suction catheter 122, the radially outer portion of the first exposed part 114 comprises a first curved portion 124 having a relatively large amount of curving deformation, whereas the radially outer portion of the distal end first lumen 106 comprises a second curved portion 126 having a relatively small amount of curving deformation.

In the first curved portion 124, the center of curvature is positioned on the lower side than the upper portion of the peripheral wall 20 of the main body shaft 18, while in the second curved portion 126, the center of curvature is positioned on the upper side than the upper portion of the peripheral wall 20 of the main body shaft 18. That is, as shown in FIG. 18, the distal end portion of the suction catheter 122 is curved roughly in an S shape. An embodiment of deformation of the control wire 54 in the case where the distal end of the control wire 54 is fixed to the lower side of the main body shaft 18 is as indicated by the chain double-dashed line in FIG. 18. Still, the embodiment of deformation in the suction catheter 122 is the same as in the case where the distal end of the control wire 54 is fixed to the upper side of the main body shaft 18.

In the suction catheter 122 of the present practical embodiment as well, the first and second exposed parts 114, 116 are exposed to the suction lumen 12. Thus, the same effect as in the first practical embodiment can be exhibited.

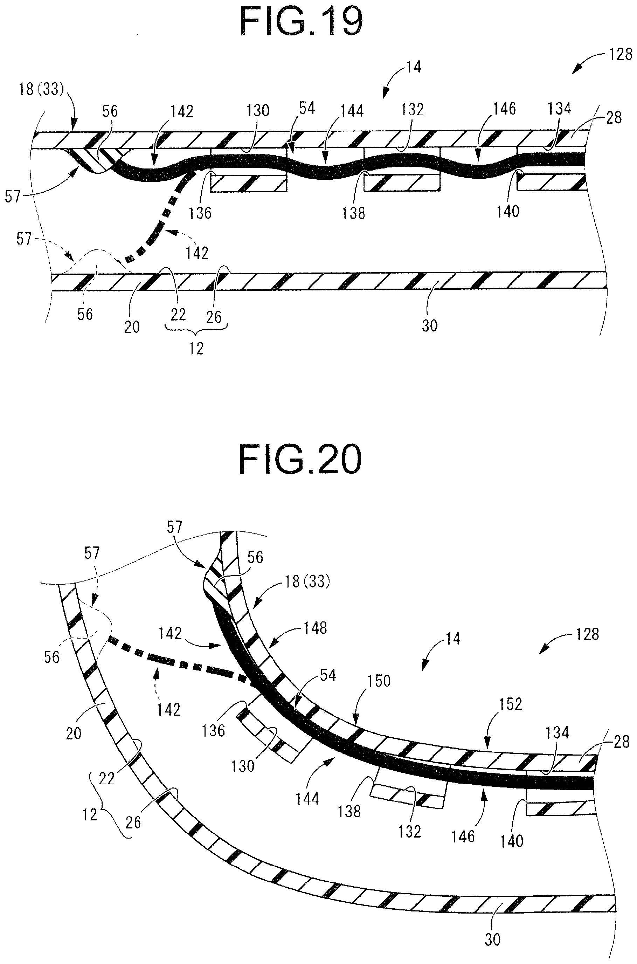

Next, FIG. 19 depicts a suction catheter 128 used for a lower-limb blood vessel as a sixth practical embodiment of the catheter according to the present invention. In the present practical embodiment as well, the illustration of the portion other than the distal end portion of the control wire 54 is omitted.

In the suction catheter 128 of the present practical embodiment, three first lumens are provided in the lengthwise direction, namely, a distal end first lumen 130, an intermediate first lumen 132, and a proximal end first lumen 134 serving as control lumens are provided. The distal end, intermediate, and proximal end first lumens 130, 132, and 134 are provided at different positions in the lengthwise direction, while being provided at the same position (upper side) on the circumference. Therefore, a part of the peripheral wall of the distal end, intermediate, and proximal end first lumens 130, 132, and 134 is each constituted by the upper portion of the peripheral wall 20 of the main body shaft 18.

The control wire 54 is inserted through each of the distal end, intermediate, and proximal end first lumens 130, 132, and 134, and the distal end of the control wire 54 is fixed to the upper side of the inner surface of the main body shaft 18 with the adhesive 56 further on the distal end side than the distal end first lumen 130. Furthermore, portions protruding from a first distal end opening 136, a second distal end opening 138, and a third distal end opening 140 serving as window parts, which are the respective distal end openings of the first lumens 130, 132, and 134, are exposed to the suction lumen 12. Besides, an embodiment in which the distal end of the control wire 54 is fixed to the lower side of the inner surface of the main body shaft 18 is indicated by the chain double-dashed line in FIG. 19.

In the suction catheter 128 of this practical embodiment having the above-described structure as well, as shown in FIG. 20, by displacing the operating part (38) toward the proximal end side, an operating force in the pulling direction is exerted on the control wire 54, and the distal end of the suction catheter 128 is deformed to curve upward. At that time, the deformation rigidity of the radially outer portions of a first exposed part 142, a second exposed part 144, and a third exposed part 146 in the main body shaft 18 is made small, thereby making curving deformation easy. That is, when the suction catheter 128 undergoes curving deformation, first, second, and third curved portions 148, 150, and 152 are configured to be formed in the main body shaft 18 correspondingly to the first, second, and third exposed parts 142, 144, and 146. The first, second, and third curved portions 148, 150, and 152 are configured such that the curvature increases in the order of the first curved portion 148, the second curved portion 150, and the third curved portion 152. This is because, by exerting an operating force in the pulling direction on the control wire 54, the stress applied to the edge parts of the window parts increases sequentially from the distal end first lumen 130. An embodiment of deformation of the control wire 54 in the case where the distal end of the control wire 54 is fixed to the lower side of the main body shaft 18 is as indicated by the chain double-dashed line in FIG. 20. The embodiment of deformation in the suction catheter 128 is the same as in the case where the distal end of the control wire 54 is fixed to the upper side of the main body shaft 18.

In the suction catheter 128 of the present practical embodiment as well, the control wire 54 is provided with the first, second, and third exposed parts 142, 144, and 146 exposed to the suction lumen 12. Thus, the same effect as in the first practical embodiment can be exhibited.

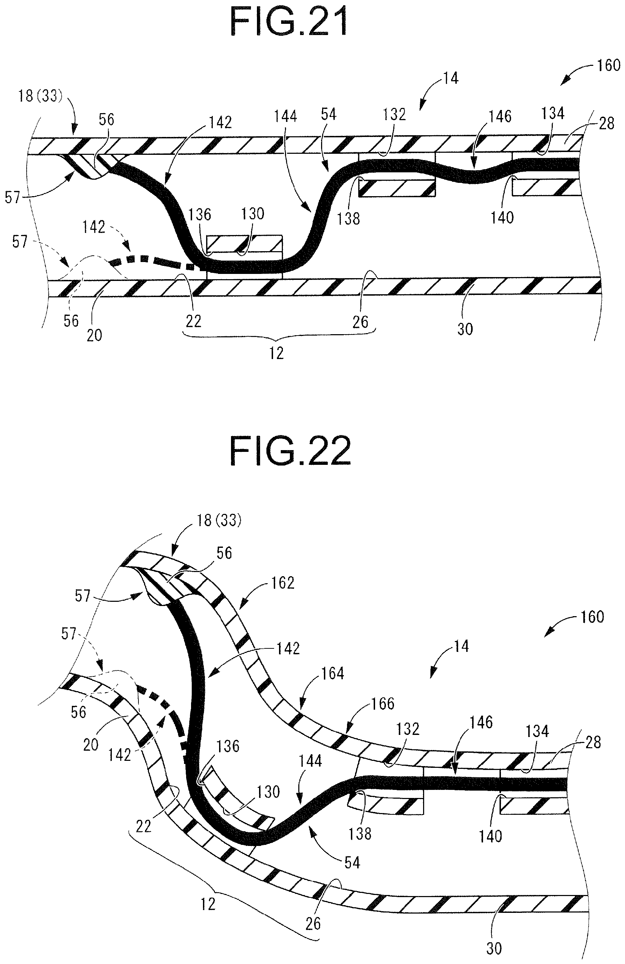

Next, FIG. 21 depicts a suction catheter 160 used for a lower-limb blood vessel as a seventh practical embodiment of the catheter according to the present invention. In the present practical embodiment as well, the illustration of the portion other than the distal end portion of the control wire 54 is omitted.

In the present practical embodiment, three first lumens (distal end, intermediate, and proximal end first lumens 130, 132, and 134) are provided similarly to the sixth practical embodiment, while the circumferential position of the distal end first lumen 130 and the circumferential position of the intermediate and proximal end first lumens 132, 134 are made different from each other. Specifically, the distal end first lumen 130 is provided at the lower portion of the main body shaft 18, and the intermediate and proximal end first lumens 132, 134 are provided at the upper portion of the main body shaft 18. That is, a part of the peripheral wall of the distal end first lumen 130 is constituted by the lower portion of the peripheral wall 20 of the main body shaft 18, while a part of the peripheral wall of the intermediate and proximal end first lumens 132, 134 is constituted by the upper portion of the peripheral wall of the main body shaft 18.

The control wire 54 is inserted through each of the distal end, intermediate, and proximal end first lumens 130, 132, and 134, and the distal end of the control wire 54 is fixed to the upper portion of the inner surface of the main body shaft 18 further on the distal end side than the distal end first lumen 130.

In addition, in the control wire 54, portions protruding from the respective distal end openings (the first, second, and third distal end openings 136, 138, and 140) of the distal end, intermediate, and proximal end first lumens 130, 132, and 134 are exposed to the suction lumen 12.

In the suction catheter 160 having the above-described structure as well, as shown in FIG. 22, by displacing the operating part (38) toward the proximal end side, an operating force in the pulling direction is exerted on the control wire 54, and the distal end of the suction catheter 160 is deformed to curve upward. At this time, in the main body shaft 18, the curving deformation easily occurs at the positions where the first and second exposed parts 142, 144 are formed, while the curving deformation is relatively less likely to occur at the position where the distal end first lumen 130 is formed. Therefore, when the suction catheter 160 deforms to curve, a first curved portion 162, a second curved portion 164, and a third curved portion 166 are configured to be provided correspondingly to the first exposed part 142, the distal end first lumen 130, and the second exposed part 146.

The center of curvature of the first curved portion 162 is positioned on the lower side than the upper peripheral wall 20 of the main body shaft 18, while the centers of curvature of the second and third curved portions 164, 166 are positioned on the upper side than the upper peripheral wall 20 of the main body shaft 18. Accordingly, the distal end portion of the suction catheter 160 is configured to deform to curve roughly in an S shape. An embodiment of deformation of the control wire 54 in the case where the distal end of the control wire 54 is fixed to the lower side of the main body shaft 18 is as indicated by the chain double-dashed line in FIG. 22. Still, the embodiment of deformation in the suction catheter 160 is the same as in the case where the distal end the control wire 54 is fixed to the upper side of the main body shaft 18.

In the present practical embodiment as well, each of the first, second and third exposed parts 142, 144, and 146 is exposed to the suction lumen 12. Thus, the same effect as in the first practical embodiment can be exhibited.

Although the practical embodiments of the present invention have been described above, the present invention is not limitedly interpreted based on the specific description in the practical embodiment, but may be embodied with various changes, modifications and improvements which may occur to those skilled in the art.

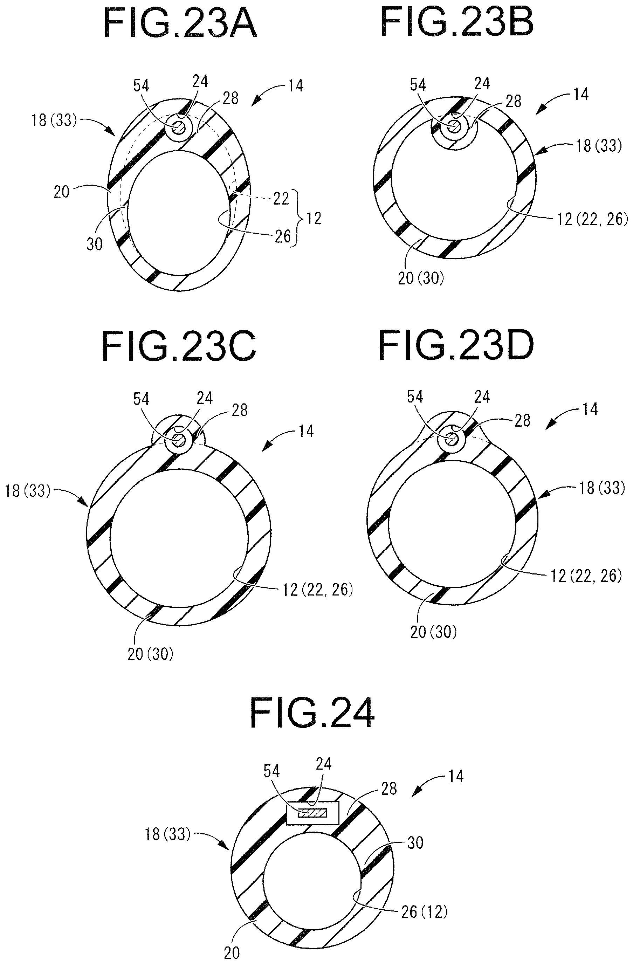

For example, in the first practical embodiment and the like, as shown in FIGS. 3 and 4, the main body shaft 18, as well as the central lumen 22, the first lumen 24, and the second lumen 26 provided inside the main body shaft 18, are all have a perfect circular shape. However, as shown in FIG. 23A, the main body shaft 18, the central lumen 22, the first lumen 24, and the second lumen 26 may have an elliptical shape, or may adopt various shapes such as a semicircular shape, a polygonal shape, or a combination thereof.

Moreover, in the preceding practical embodiment, the first lumen 24 and the second lumen 26 are provided in parallel in the vertical direction. However, as shown in FIG. 23B for example, the first lumen 24 may be provided inside the second lumen 26 (the main lumen). That is, the peripheral wall 28 of the first lumen 24 may be provided so as to protrude into the inside of the peripheral wall 20 of the main body shaft 18. Alternatively, as shown in FIG. 23C, the peripheral wall 28 of the first lumen 24 may be provided so as to protrude to the outside of the peripheral wall 20 of the main body shaft 18. In such a case, as shown in FIG. 23D, the radially outer surface of the peripheral wall 20 of the main body shaft 18 and the radially outer surface of the peripheral wall 28 of the first lumen 24 may be connected with a smooth curve.

Furthermore, the cross-sectional shape of the shaft member (the main body shaft), the central lumen, the first lumen, and the second lumen need not be constant in the lengthwise direction, but may be varied in the lengthwise direction by combining a plurality of the above-described shapes, and the like.

Besides, as shown in FIG. 24, the cross section of the control wire 54 may be rectangular. Indeed, the cross-sectional shape of the control wire is not limited to the circular shape as in the preceding practical embodiment or the rectangular shape shown in FIG. 24. Various shapes such as an ellipse, a semicircle, a triangle or a polygon with five or more sides, or a combination thereof may be adopted. In addition, the cross-sectional shape of the control wire need not be constant in the lengthwise direction, but may be varied in the lengthwise direction by gradually decreasing its diameter toward the distal end, by combining a plurality of the above-described shapes, and the like, for example. As shown in FIG. 24, the cross-sectional shape of the control lumen (the first lumen) 24 may be similar to or different from the cross-sectional shape of the control wire 54.

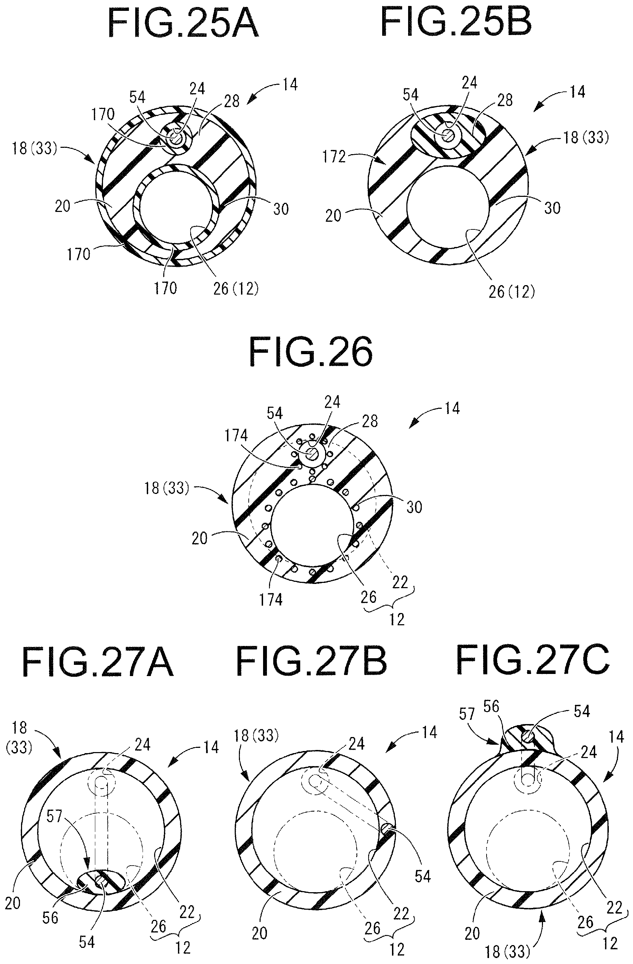

Additionally, in the preceding practical embodiment, the peripheral wall 20 of the main body shaft 18 is made of the same material, but the material may be partially different. Specifically, for example, as shown in FIG. 25A, on the radially outer surface of the peripheral wall 20 of the main body shaft 18, the radially inner surface of the first lumen 24, and the radially inner surface of the second lumen 26 (the suction lumen 12), a coating layer 170 may be formed of a material having a small friction coefficient such as polytetrafluoroethylene (PTFE) or the like. This makes it possible to reduce the sliding resistance when the control wire 54 slides within the first lumen 24, for example, and to reduce the sliding resistance when the main body shaft 18 slides within the blood vessel, and the like. However, such a coating layer is not limited to those having a small friction coefficient, but the material of the coating layer can be made different according to the required characteristics. The layer having a small friction coefficient as described above can be formed not only by coating the main body shaft 18. For example, it would also be acceptable that a tubular body formed of a material having a small friction coefficient is inserted into or placed externally about the main body shaft 18 so as to be secured to each other.

Further, as described above, when the material of the shaft member is partially made different, it is not necessary to form a thin layer like a coating layer, but may give a predetermined thickness dimension. That is, as shown in FIG. 25B, the radially inner surface of the first lumen 24 may be formed of a material different from that of a main body 172 of the main body shaft 18 with a predetermined thickness dimension. The main body 172 means a portion of the main body shaft 18 other than the peripheral wall constituting the radially inner surface of the first lumen 24. In such a case as well, as shown in FIG. 25B, the cross-sectional shape of the first lumen 24 need not be circular but various shapes such as an elliptical shape can be adopted. Besides, in the case where the material of the radially inner surface of the second lumen 26 is different from the material of the main body of the main body shaft 18, the main body means a portion of the main body shaft 18 other than the peripheral wall constituting the radially inner surface of the second lumen 26.

Furthermore, as shown in FIG. 26, the peripheral wall 28 of the first lumen 24, the peripheral wall 30 of the second lumen 26, and the like may be reinforced by a braided wire 174, which is made of a metal or a rigid synthetic resin, being embedded as an intermediate layer thereof. The braided wire 174 may be constituted by, for example, braiding one or a plurality of linear bodies into a mesh tubular shape, or the like.

In the first practical embodiment and the like, the distal end of the control wire 54 is fixed on the inner surface of the main body shaft 18 at the circumferentially same position (the upper side) as the control lumen 24. However, it may be fixed on the circumferentially opposite side (the lower side) as shown in FIG. 27A, or at the position circumferentially shifted by 90° or 270° (the right side or left side in FIG. 27B) as shown in FIG. 27B. In addition, the fixed position may be inside the peripheral wall 20 as shown in FIG. 27B, or may be outside the peripheral wall 20 as shown in FIG. 27C, other than on the inner surface of the main body shaft 18 as in the preceding practical embodiment. The method for fixing the distal end of the control wire to the shaft member is not limited to bonding with the adhesive as in the preceding practical embodiment, but may be welding, bonding by pressure, or a mechanical connection such as caulking, hooking or pinching, and the like. Further, the adhesive may contain a metal body such as an imaging marker showing radiopacity, for example.

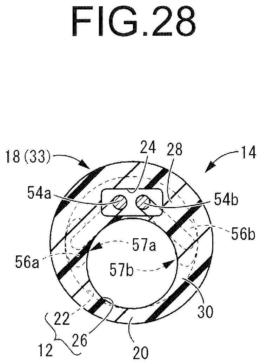

Moreover, in the third practical embodiment, the two first lumens 24a, 24b are provided and the control wires 54a, 54b are inserted therethrough respectively. However, as shown in FIG. 28, the two control wires 54a, 54b may be inserted through a single first lumen 24. It should be appreciated that the control wires 54a and 54b in the third practical embodiment need not be independent of each other. For example, the proximal ends of the control wires 54a, 54b may be connected to each other so as to be constituted by a single control wire. In such a case, it is possible to simultaneously perform the pulling operation (the pushing operation) of the control wire 54a and the pushing operation (the pulling operation) of the control wire 54b.

Furthermore, whereas in the first practical embodiment and the like, the window part exposing the control wire 54 to the suction lumen (the main lumen) 12 is constituted by the distal end opening 58 of the first lumen (the control lumen) 24, the present invention is not limited to such an embodiment. That is, the window part may be provided to the peripheral wall of the control lumen, and the control wire may be exposed through the window part to the main lumen, for example. Therefore, the distal end of the control lumen need not be open, and when the distal end of the control lumen is closed, the distal end of the control wire may be fixed to the inner surface of the peripheral wall of the control lumen, for example.

In the first practical embodiment and the like, the suction catheter comprises an over-the-wire type catheter. However, by providing a guide wire lumen that opens at the distal end and the radially outer surface of the shaft member separately from the suction lumen, the catheter may also be a rapid-exchange type.

Besides, the catheter according to the present invention is not limited to a suction catheter as described in the preceding practical embodiment, a balloon catheter, or the like. That is, with the catheter according to the present invention, there is a case of inserting the catheter into a somatic lumen while checking the distal end portion thereof under the X-ray fluoroscopy or the like, for example. By operating the operating part, it is possible to deform the distal end portion to curve so as to conform to the curved shape and the branched shape of the somatic lumen, thereby reducing the risk of damage to the somatic lumen or the like.

With the catheter according to the present invention, in the initial state before exerting an operating force on the control wire, it is not necessary for the catheter main body (the shaft member) to extend linearly as shown in FIG. 1 and the like. Specifically, it would also be acceptable that the catheter main body (the shaft member) has a shape of curving or bending downward in the initial state, for example, and by operating the control wire, the shaft member bends upward so that the catheter main body becomes linear, or bends further upward. According to such a catheter, in comparison with the case where the operating forces in both the pulling direction and the pushing direction are exerted on the control wire as described in the first practical embodiment and the like, the catheter can be operated simply by exerting the operating force in the pulling direction, for example. This makes it possible not only to further decrease the diameter of the control wire, but also to further simplify the structure of the controller.

### KEYS TO SYMBOLS

10, 62, 98, 100, 104, 122, 128, 160: suction catheter used for a lower-limb blood vessel, 12, 76: suction lumen (separate internal space, main lumen, separate lumen), 16, 102: controller, 18: main body shaft, 24, 24a, 24b, 70: first lumen (control lumen), 26, 72: second lumen, 28, 80: peripheral wall (peripheral wall of the control lumen), 33, 78: shaft member, 54, 54a, 54b, 64: control wire, 57, 57a, 57b: fixing part, 58, 58a, 58b, 88: distal end opening (window part), 60, 60a, 60b, 96: exposed part, 86, 174: braided wire, 106, 130: distal end first lumen (control lumen), 108, 134: proximal end first lumen (control lumen), 110, 136: first distal end opening (window part), 112, 138: second distal end opening (window part), 114, 142: first exposed part, 116, 144: second exposed part, 132: intermediate first lumen, 140: third distal end opening (window part), 146: third exposed part, 172: main body

## Claims

1. A catheter comprising:
an elongated shaft member including at least one control lumen extending in a lengthwise direction;
at least one control wire inserted through the control lumen; and
at least one fixing part to which a distal end of the control wire is fixed, the fixing part being provided to the shaft member, wherein
at least one window part is provided to a peripheral wall of the control lumen, and
an exposed part is provided to a distal end portion of the control wire over a predetermined length, the exposed part being exposed through the window part to a separate internal space in the shaft member.

2. The catheter according to claim 1, wherein the separate internal space comprises a separate lumen extending in the lengthwise direction of the shaft member.

3. The catheter according to claim 2, wherein the separate lumen comprises a main lumen extending with a cross section larger than that of the control lumen.

4. The catheter according to any one of claims 1-3, wherein the window part is provided at a position away from the fixing part of the shaft member by 10 to 50 mm.

5. The catheter according to any one of claims 1-4, wherein the fixing part is provided at a position away from a distal end of the shaft member by 10 to 40 mm.

6. The catheter according to any one of claims 1-5, further comprising a controller provided on a proximal end side of the control wire, the controller being configured to exert an operating force in the lengthwise direction from an outside.

7. The catheter according to any one of claims 1-6, wherein the shaft member is reinforced by a braided wire embedded as an intermediate layer thereof.

8. The catheter according to any one of claims 1-7, wherein a radially inner surface of either one of the control lumen and the separate internal space is formed of a material different from that of a main body of the shaft member.

9. The catheter according to any one of claims 1-8, wherein the window part is provided further on a proximal end side than the fixing part in the lengthwise direction of the shaft member.

10. The catheter according to any one of claims 1-8, wherein the window part is provided further on a distal end side than the fixing part in the lengthwise direction of the shaft member.

11. The catheter according to any one of claims 1-10, wherein
the at least one control wire comprises a plurality of control wires,
the at least one fixing part comprises a plurality of fixing parts provided correspondingly to the plurality of control wires, and
positions of the fixing parts are different from each other in a circumferential direction.

12. The catheter according to claim 11, wherein the at least one control lumen through which the control wires are inserted comprises a plurality of control lumens provided correspondingly to the plurality of control wires.

13. The catheter according to any one of claims 1-12, wherein the at least one window part comprises a plurality of window parts spaced away from each other in the lengthwise direction of the shaft member.

14. The catheter according to any one of claims 1-13, wherein the at least one control lumen comprises a plurality of control lumens provided at different positions in either one of a circumferential direction and the lengthwise direction of the shaft member.

15. The catheter according to any one of claims 1-14, wherein the catheter comprises a suction catheter for suctioning a thrombus and the like in a blood vessel.

16. The catheter according to claim 15, wherein the suction catheter is used for a lower-limb blood vessel.
